# EUROPEAN PATENT SPECIFICATION

(11) **EP 0 832 622 B1**
(45) Date of publication and mention of the grant of the patent: **05.03.2003**
(21) Application number: 97115877.9
(22) Date of filing: 12.09.1997
(51) Int. Cl.: A61F 2/44, A61F 2/46

(54) **A spinal cage assembly**
Wirbelkäfigsatz
Ensemble de cage vertébrale

(30) Priority: 26.09.1996 DE 29616778 U
(43) Date of publication of application: 01.04.1998
(73) Proprietor: Stryker Trauma GmbH, 24232 Schönkirchen (DE)
(72) Inventor: Metz-Stavenhagen, Peter, Dr., 34537 Bad Wildungen (DE); Robioneck, Bernd, 24111 Preetz (DE)
(74) Representative: Patentanwälte Hauck, Graalfs, Wehnert, Döring, Siemons, Schildberg

(56) References cited:
- EP-A- 0 188 954
- EP-A- 0 268 115
- WO-A-90/00037
- WO-A-94/18913
- DE-A- 4 409 392
- DE-A- 4 423 257
- DE-A- 19 509 317
- DE-A- 19 519 101
- DE-C- 4 323 034
- DE-C- 19 500 170
- DE-C- 19 504 867
- DE-U- 29 616 778
- US-A- 5 015 247

## Description

The present invention relates to a spinal cage assembly.

Spinal implants have become known which are formed as hollow members and which are inserted - individually or in pairs - into the area between adjacent vertebrae in order to obtain effective adherence between adjacent vertebrae. Such implants are shown for example in European patent 0 307 741 B1, German patents 44 16 605 C1 and 43 23 956 C1, European patent application 0 637 440 A1, PCT-application WO 95/26164, US patent 5,015,247 and German utility model G 296 00 879. The hollow structure of the implants enables receival of bone material or the like for enhancing bone growth and adherence.

Furthermore so-called spinal cage assemblies have become known which are implanted for example after removal of a vertebra so as to replace the removed vertebra. Such spinal cage assemblies have become known for example from German utility model 91 07 494 and European patent applications 0 188 054 and 0 535 215. Such prior spinal cage assemblies engage the vertebrae in a substantially central area of relatively soft structure. Therefore, there is a risk of the spinal cage assembly "digging" into the vertebra.

A similar risk is present with spinal cage assemblies made of relatively thin wire mesh.

An object of the invention is to provide a spinal cage assembly allowing for effective support against and enabling growth of new bone substance between adjacent vertebrae.

To this end the invention provides a spinal cage assembly including a sleeve-shaped member of a metal compatible with the human body, said sleeve-shaped member having end faces defining irregular edges and having a wall provided with holes.

The spinal cage assembly of the present invention comprises a sleeve-shaped member of a material compatible with the human body. The sleeve-shaped member has a relatively thick wall suitable to receive and transfer substantial forces and enabling engagement with adjacent vertebrae under relatively small surface pressure. The support provided thereby is particularly effective when the diameter of the sleeve-shaped member is dimensioned such that the wall engages the vertebra in the cortical area thereof. As is known, the cortex is the hardest bone area.

In accordance with the invention the end faces or face edges of the sleeve-shaped member are of irregular shape. This prevents the sleeve-shaped member from rotary movements. Furthermore the wall of the sleeve-shaped member includes windows through which bone material may be inserted into the interior of the sleeve-shaped member.

Preferably, the irregular edge of the sleeve-shaped member extends in a plane which is inclined to a transverse axis of the sleeve-shaped member under a predetermined angle of e.g. 3 to 6°. This allows for adaption to different anatomic structures.

The end face of the sleeve shaped member may be provided with teeth and gaps therebetween, with the tips of the teeth being formed as planar surfaces such as to prevent the spinal cage assembly from digging into the vertebrae. Preferably, the gaps are of a round contour, with the gaps and the top surfaces of the teeth forming relatively sharp edges.

For assembly of a sleeve-shaped member a tool may be used, which has a relatively long shank and includes a threaded extension at a free end thereof. The threaded extension may be engaged into a threaded bore of the sleeve-shaped member in order to insert the latter into the space of the removed vertebra in a predetermined position between two adjacent vertebrae.

The height of the spinal cage assembly depends on the height of the removed vertebra or, respectively, the spacing between the remaining adjacent vertebrae, and on the position of the vertebra in the spine. Therefore, the prior art spinal cage assemblies generally are adjustable in height. In order to enable adjustments also with the spinal cage assembly of the present invention, it is preferred to provide a second sleeve-shaped member telescopically receiving the first sleeve-shaped member and being of similar shape as the internal sleeve-shaped member. Furthermore, both sleeve-shaped members include bores adapted to be aligned to each other and to receive threaded bolts for holding the two sleeve-shaped members stationary with respect to each other. When in this position the internal sleeve-shaped member projects somewhat from the external sleeve-shaped member. When a pair of bores is provided, two different relative positions may be set. Together with the height of the internal sleeve-shaped member there will result four different heights which will be completely sufficient for most of the cases occurring in practice. The height of the spinal cage assembly is adjusted before the implant is implanted so that difficult manipulations for height adjustment in situ are not required.

According to another aspect of the present invention a force member or the like may be mounted between the sleeve-shaped members telescopically received in each other in order to provide for automatic height adjustment. For example a biased spring may be provided to move the sleeve-shaped members apart from each other when a respective fixing means will be released. Other force members may be used.

While the sleeve-shaped member may be of square or polygonal cross-section, the circular cross-section in conformity with the contour of the vertebrae is preferred.

As already mentioned the tool for implanting the sleeve-shaped member has a relatively long shank. Such shank may rotatably receive a sleeve at its free end, which sleeve engages a shoulder of the shank and has at its free end a concave recess of a shape conforming to the shape of the external contour of the sleeve-shaped member. In this manner transverse forces to be applied by the tool when the sleeve-shaped member is being implanted, are transferred not only via the relatively thin threaded extension but also via the sleeve.

The invention will be explained in more detail with reference to drawings.
Fig. 1 is a perspective view of a spinal cage assembly according to the invention;
Fig. 2 is a side elevation of the spinal cage assembly of Fig. 1 in a first direction;
Fig. 3 is a side elevation of the spinal cage assembly of Fig. 1 in the opposite direction;
Fig. 4 is an elevation of the spinal cage assembly of Fig. 1 in the assembled condition;
Fig. 5 is a side elevation of the internal sleeve-shaped member of the spinal cage assembly of Fig. 1;
Fig. 6 shows schematically a tool for handling the spinal cage assembly of Fig. 1;
Fig. 7 shows a detail in Fig. 6;
Fig. 8 is a cross-section of the sleeve of the tool of Fig. 6.

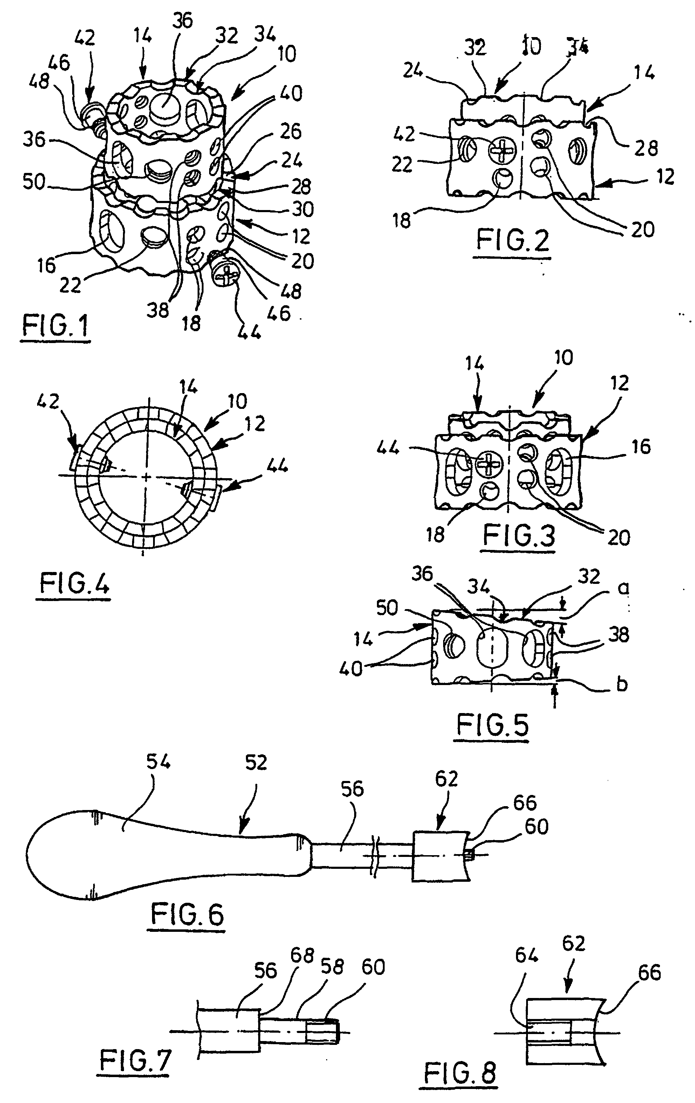

A spinal cage assembly 10 as shown in Figs. 1 to 4 comprises an external sleeve-shaped member 12 and an internal sleeve-shaped member 14. Both sleeve-shaped members 12, 14 have a relative thick wall, are made of a metal compatible with the human body, preferably titanium, and are of a circular cross-section. The dimensions are such that the sleeve-shaped member 14 is telescopically received in the external sleeve-shaped member 12. Furthermore the diameters of the external sleeve-shaped member 12 and the internal sleeve-shaped member 14 are selected such that the spinal cage assembly is supported substantially against the cortex when it is positioned between adjacent vertebrae. The shown spinal cage assembly is suited primarily for lumbal vertebrae.

The external sleeve-shaped member 12 includes a pair of elongated windows shown at 16 in Figs. 1 and 3. They can be used for inserting bone material.

The external sleeve-shaped member includes further eight radial holes 18 and 20 positioned in pairs one above the other. The holes of one pair are positioned above each other along an axis parallel to the longitudinal axis of the sleeve-shaped member 12. The holes of adjacent pairs 18, 20 are offset with respect to each other as may be best seen in Figs. 2 and 3.

Finally the sleeve-shaped member 12 includes a pair of threaded bores one of which is shown at 22. The purpose of the various holes or bores in the wall of the sleeve-shaped member 12 will be explained in more detail below.

The sleeve-shaped member 12 has at its upper and lower sides circumferentially uniformly spaced teeth 24 having flat upper surfaces 26. Gaps 28 of circular arc-shape are provided between the teeth and define together with the teeth relatively sharp edges in the area of the upper surfaces 26. Therefore, the upper and lower sides of the sleeve-shaped member prevent rotary movements thereof when it is implanted so as to provide a rotation preventing means.

The internal sleeve-shaped member 14 is of similar shape as member 12 and has upper and lower sides provided with teeth 32 and rounded gaps 34. The internal sleeve-shaped member 14 includes also pairs of windows 36 which are of elongated shape and can be aligned to the windows 16. Furthermore, the internal sleeve-shaped member 14 includes four threaded bores positioned in pairs one above each other, of which two pairs 38, 40 may be seen in Fig. 2. The bores of each pair are positioned vertically above each other. The bores of each pair may be aligned to the holes of a pair 18, 20 of the external sleeve-shaped member 12. Two screws or threaded bolts 42, 44 may be inserted into the holes 18 and 20, respectively. A smooth portion 46 thereof will be positioned in a hole 18 or, respectively, 20, while a threaded portion 48 may be engaged into the respective threaded bore 38 or 40, respectively, in order to hold the internal and external sleeve-shaped members 14, 12 stationary with respect to each other as shown in Fig. 4. Since the holes or bores of the pairs 18, 20 and, respectively, the pairs 38, 40 are offset with respect to each other in height, different relative positions of the internal and external sleeve-shaped members may be set. When for example the lowest bore of the pair 38 is aligned with the lowest hole of pair 18, the internal sleeve-shaped member 14 projects only slightly from the external sleeve-shaped member 12. When, however, the upper bore of pair 40 is aligned to the upper hole of pair 20, the internal sleeve-shaped member 14 projects maximally from the external sleeve-shaped member 12.

The internal sleeve-shaped member 14 includes further threaded bores 50 of which one is shown in Fig. 1 and two are shown in Fig. 2. The threaded bores 50 serve - in the same manner as threaded bores 22 - for connection with a tool shown in Figs. 6 to 8 as will be explained in more detail below.

The internal sleeve-shaped member 14 has an inclined upper surface as shown in Fig. 3. Fig. 5 shows the internal sleeve-shaped member 14 separately. As may be seen the upper surface is inclined to a transverse axis under an angle a of 6° and the lower surface under an angle b of 3°. Depending on which end of the internal sleeve-shaped member 14 is inserted first into the external sleeve-shaped member, a respective inclination of the upper surface will result. This allows for adaptation to respective anatomic geometries.

It will be appreciated that an internal sleeve-shaped member 14 or an external sleeve-shaped member 12 alone may be used.

Figs. 6 to 8 show a suitable assembly tool 52 having a handle 54, a shaft 56 of a length of e.g. 20 cm and an extension 58 of a reduced diameter at the forward end. As is apparent from Fig. 7 the extension 58 has a threaded portion 60 at its free end. A sleeve 62 is mounted on the extension and has a bore with an internal threaded portion 64 extending to the left hand end while being spaced from the right hand end. The right hand or free end of the sleeve 62 has a concave recess 66 which is of a shape conforming to the round contour of the internal sleeve-shaped member 14 or the external sleeve-shaped member 12. During assembly, the sleeve 76 is threadingly engaged on the threaded portion 60 of the extension 58 and will then have one end engage a respective shoulder 68 of shaft 56. As a result, the sleeve 62 is mounted for rotation, however, fixed against axial displacement by the threads and may be removed only by unscrewing. When the tool will be connected to the spinal cage assembly of Figs. 1 to 5, the threaded portion 60 of the extension 58 will be threaded into the threaded bore 22 or threaded bore 50, with the sleeve 62 being rotated such that it will matingly engage the external side of the sleeve wall. This allows to transmit forces from shank 56 to the spinal cage assembly.

## Claims

1. A spinal cage assembly comprising an external sleeve member (12) and an internal sleeve member (14) both of metal compatible with the human body, the external sleeve member (12) being adapted to telescopically receive the internal sleeve member (12), the sleeve members (12, 14) having end faces defining irregular edges, the sleeve members including bores or holes (18, 20, 38, 40) to be aligned with each other and to receive threaded bolts (44, 46) for holding the sleeve members (12, 14) stationary with each other in different height positions.

2. The spinal cage assembly of claim 1, wherein at least one irregular edge of at least one of sleeve members (12, 14) is inclined with respect to a transverse axis of the sleeve members (12, 14) under a predetermined angle.

3. The spinal cage assembly of claim 2, wherein the predetermined angle is between 3° and 6°.

4. The spinal cage assembly of one of the claims 1 to 3, wherein the irregular edges are provided with teeth (24, 32) and gaps (28, 34) therebetween.

5. The spinal cage assembly of claim 1, wherein the teeth (24, 32) have tips formed as a planar surface (26).

6. The spinal cage assembly of claim 4, wherein the gaps (28, 34) are of circular arc shape, the gaps and said planar surfaces (26) defining relatively sharp edges therebetween.

7. The spinal cage assembly of one of the claims 1 to 6, wherein the walls of the sleeve members (12, 14) include elongated holes (16, 36) having longitudinal axes substantially parallel to a longitudinal axis of sleeve members (12, 14).

8. The spinal cage assembly of one of the claims 1 to 7, wherein the wall of the external sleeve member (12) includes at least one threaded bore (22, 50) for receiving a positioning tool (52) which is provided with a threaded extension (58, 60) at a free end thereof.

9. The spinal cage assembly of one of the claims 1 to 8, wherein the external sleeve member (12) includes at least two circumferentially spaced holes (18, 20), the internal sleeve member (14) including at least two circumferentially spaced threaded bores (38, 40) adapted to be aligned to the radial holes (18, 20) of the external sleeve member (12) such that the internal sleeve member (14) partially projects from the external sleeve member (12).

10. The spinal cage assembly of claim 9, wherein two or three pairs of radial holes (18, 20) and threaded bores (38, 40) are provided, the holes and, respectively, bores of each pair being disposed above each other along an axis parallel to a longitudinal axis of the external sleeve member (12) or, respectively, internal sleeve member (14).

11. The spinal cage assembly of one of the claims 1 to 10, wherein a force member acts between the external and internal sleeve member so as to enable adjustment thereof in height, and fixing means are provided to fix the sleeve-shaped members in their respective positions.

12. The spinal cage assembly of one of the claims 1 to 11, wherein the walls of the sleeve members (12, 14) are provided with radial windows (16).

13. The spinal cage assembly of one of the claims 1 to 12, wherein the external and the internal sleeve members (14, 12) are of circular cross section.

14. The spinal cage assembly of one of the claims 1 to 13, wherein the threaded bolts (42, 44) comprise a head and a stem, the stem comprising a threaded portion (48) and a cylindrical portion (46) arranged between said threaded portion and the head and being of a diameter corresponding substantially to the diameter of the radial holes (18, 20) of the external sleeve member (12)

15. The combination of a tool and the spinal cage assembly as defined in one of the claims 8 to 14, wherein the tool has an elongated shank (56) having a handle (54) at one end thereof and a threaded extension (60) at the other end.

16. The combination of claim 17, wherein a sleeve (62) is rotatably mounted upon said threaded extension (68) so as to cooperate with a shoulder (68) of said elongated shaft (56) at an end thereof remote from said threaded extension (60), and includes a concave recess (66) of a shape conforming to the rounded shape of said internal or, respectively, external sleeve-shaped member (14, 12).

17. The combination as defined in claim 16, wherein said threaded extension (60) includes a smooth portion (58) and said sleeve (62) has an internal threaded portion (64) spaced from a free end thereof, said internal threaded portion (64) being out of engagement with the threads of said threaded extension (60) when said sleeve (62) engages said shoulder (68)

## Patentansprüche

1. Wirbelkörperplatzhalter mit einem äußeren hülsenförmigen Körper (12) und einem inneren hülsenförmigen Körper (14), beide aus körperverträglichem Metall, wobei der äußere hülsenförmige Körper (12) ausgelegt ist, um den inneren hülsenförmigen Körper (14) teleskopisch aufzunehmen, wobei die hülsenförmigen Körper (12, 14) Stirnenden besitzen, die unregelmäßige Kanten bilden, wobei die hülsenförmigen Körper (12, 14) Bohrungen oder Löcher (18, 20, 38, 40) aufweisen, um miteinander ausgerichtet zu werden und Gewindeschrauben (44, 46) aufzunehmen, um die hülsenförmigen Elemente (12, 14) gegeneinander in unterschiedlichen Höhenpositionen festzulegen.

2. Wirbelkörperplatzhalter nach Anspruch 1, **dadurch gekennzeichnet, daß** mindestens eine unregelmäßige Kante an mindestens einem hülsenförmigen Element (12, 14) in einem vorbestimmten Winkel zur Querachse des hülsenförmigen Körpers (12, 14) verläuft.

3. Wirbelkörperplatzhalter nach Anspruch 2, **dadurch gekennzeichnet, daß** der vorbestimmte Winkel zwischen 3° und 6° beträgt.

4. Wirbelkörperplatzhalter nach einem der Ansprüche 1 bis 3, **dadurch gekennzeichnet, daß** die unregelmäßige Kante mit Zähnen (24, 32) und Zahnlücken (28, 34) geformt ist.

5. Wirbelkörperplatzhalter nach Anspruch 4, **dadurch gekennzeichnet, daß** die Zähne (24, 32) an der Spitze plane Flächen (26) aufweisen.

6. Wirbelkörperplatzhalter nach Anspruch 4, **dadurch gekennzeichnet, daß** die Zahnlücken (28, 34) kreisbodenförmig gerundet sind, wobei die Zahnlücken mit den planen Flächen (26) relativ scharfe Kanten bilden.

7. Wirbelkörperplatzhalter nach einem der Ansprüche 1 bis 6, **dadurch gekennzeichnet, daß** Langlöcher (16, 36) in der Wandung der hülsenförmigen Körper geformt sind, deren Längsachse annähernd parallel zur Längsachse des hülsenförmigen Körpers (12, 14) verläuft.

8. Wirbelkörperplatzhalter nach einem der Ansprüche 1 bis 7, **dadurch gekennzeichnet, daß** in der Wandung mindestens ein Gewindeloch (22, 50) geformt ist für den Einsatz eines Positionierwerkzeugs (52), das am freien Ende einen Gewindezapfen (58, 60) aufweist.

9. Wirbelkörperplatzhalter nach einem der Ansprüche 1 bis 8 **gekennzeichnet dadurch, daß** der äußere hülsenförmige Körper (12) mindestens zwei radiale Löcher (18, 20) aufweist, der innere hülsenförmige Körper (14) mindestens zwei entlang dem Umfang beabstandete Gewindelöcher (38, 40) aufweist, die mit den radialen Löchern (18, 20) des äußeren hülsenförmigen Körpers (12) übereinander angeordnet werden können derart, daß der innere hülsenförmige Körper (14) teilweise aus dem äußeren hülsenförmigen Körper (12) vorsteht.

10. Wirbelkörperplatzhalter nach Anspruch 9, **dadurch gekennzeichnet, daß** zwei oder drei Paar radiale Löcher (18, 20) bzw. Gewindebohrungen (38, 40) vorgesehen sind, wobei bei jedem Paar Loch bzw. Bohrung entlang einer Achse parallel zur Längsachse des äußeren hülsenförmigen Körpers (12) bzw. des inneren hülsenförmigen Körpers (14) übereinander angeordnet sind.

11. Wirbelkörperplatzhalter nach einem der Ansprüche 1 bis 10, **dadurch gekennzeichnet, daß** zwischen den äußeren und inneren hülsenförmigen Körpern ein Kraftglied wirkt, um diese in der Höhe zu verstellen, und Feststellmittel vorgesehen sind, um die hülsenförmigen Körper in der jeweiligen Position festzulegen.

12. Wirbelkörperplatzhalter nach einem der Ansprüche 1 bis 11, **dadurch gekennzeichnet, daß** die Wandungen der hülsenförmigen Körper (12, 14) mit radialen Durchbrechungen (16) versehen sind.

13. Wirbelkörperplatzhalter nach einem der Ansprüche 1 bis 12, **dadurch gekennzeichnet, daß** innerer und äußerer Körper (12, 14) im Querschnitt kreisförmig sind.

14. Wirbelkörperplatzhalter nach einem der Ansprüche 1 bis 13, **dadurch gekennzeichnet, daß** die Gewindeschrauben (42, 44) einen Kopf und einen Schaft aufweisen, und zwischen Kopf und Gewindeabschnitt (48) ein zylindrischer Abschnitt (46) geformt ist, dessen Durchmesser etwa dem Durchmesser der radialen Löcher (18, 20) im äußeren hülsenförmigen Körper (12) ist.

15. Kombination aus einem Werkzeug und dem Wirbelkörperplatzhalter nach einem der Ansprüche 8 bis 14, **dadurch gekennzeichnet, daß** das Werkzeug einen länglichen Schaft (56) aufweist, an dessen einem Ende eine Handhabe (54) und an dessen anderem Ende ein Gewindezapfen (60) angeordnet ist.

16. Kombination nach Anspruch 15, **dadurch gekennzeichnet, daß** auf dem Gewindezapfen (68) eine Hülse (62) drehbar gelagert ist, die an dem dem Gewindezapfen (60) entgegengesetzten Ende mit einer Schulter (68) des Schaftes (56) zusammenwirkt und am freien Ende an der Stirnfläche eine konkave Ausnehmung (66) aufweist, die an die Rundung des inneren bzw. äußeren hülsenförmigen Körpers (14, 12) angepaßt ist.

17. Kombination nach Anspruch 16, **dadurch gekennzeichnet, daß** der Gewindezapfen einen gewindefreien Abschnitt (58) aufweist, die Hülse im Abstand zum freien Ende einen Innengewindeabschnitt (64) aufweist, der bei Anlage der Hülse (62) an der Schulter (68) das Gewinde (60) des Zapfens frei läßt.

## Revendications

1. Cage spinale comportant une douille externe (12) et une douille interne (14), toutes les deux en métal compatible avec le corps humain, la douille externe (12) étant adaptée pour recevoir de façon télescopique la douille interne (12), les douilles (12, 14) ayant des faces d'extrémité définissant des bords irréguliers, les douilles comprenant des alésages ou trous (18, 20, 38, 40) devant être alignés entre eux et devant recevoir des boulons filetés (44, 46) pour maintenir les douilles (12, 14) stationnaires l'une par rapport à l'autre à différentes hauteurs.

2. Cage spinale selon la revendication 1, dans laquelle au moins un bord irrégulier d'au moins une douille (12, 14) est incliné par rapport à un axe transversal des douilles (12, 14) selon un angle prédéterminé.

3. Cage spinale selon la revendication 2, dans laquelle la valeur de l'angle prédéterminé est entre 3° et 6°.

4. Cage spinale selon l'une des revendications 1 à 3, dans laquelle les bords irréguliers sont munis de dents (24, 32) et d'espaces (28, 34) entre elles.

5. Cage spinale selon la revendication 1, dans laquelle les dents (24, 32) ont des pointes ayant une forme de surface plane (26).

6. Cage spinale selon la revendication 4, dans laquelle les espaces (28, 34) ont la forme d'arcs circulaires, les espaces et lesdites surfaces planes (26) définissant ici des bords relativement coupants.

7. Cage spinale selon l'une des revendications 1 à 6, dans laquelle les parois des douilles (12, 14) comprennent des trous oblongs (16, 36) ayant des axes longitudinaux globalement parallèles à un axe longitudinal des douilles (12, 14).

8. Cage spinale selon l'une des revendications 1 à 7, dans laquelle la paroi de la douille externe (12) comprend au moins un alésage fileté (22, 50) pour recevoir un outil de positionnement (52) qui est muni d'une extension filetée (58, 60) à l'une de ses extrémités.

9. Cage spinale selon l'une des revendications 1 à 8, dans laquelle la douille externe (12) comprend au moins deux trous répartis sur la circonférence (18, 20), la douille interne (14) comprenant au moins deux alésages filetés répartis sur la circonférence (38, 40) adaptés pour être alignés sur les trous radiaux (18, 20) de la douille externe (12) de telle façon que la douille interne (14) fait partiellement saillie de la douille externe (12).

10. Cage spinale selon la revendication 9, dans laquelle deux ou trois paires de trous radiaux (18, 20) et d'alésages filetés (38, 40) sont pratiquées, les trous et, respectivement, les alésages de chaque paire étant disposés au-dessus l'un de l'autre le long d'un axe respectivement parallèle à un axe longitudinal de la douille externe (12) ou de la douille interne (14).

11. Cage spinale selon l'une des revendications 1 à 10, dans laquelle une pièce de force agit entre les douilles externe et interne afin de permettre ici des ajustements en hauteur, et des moyens de fixation sont fournis pour bloquer les pièces en forme de douille en leurs positions respectives.

12. Cage spinale selon l'une des revendications 1 à 11, dans laquelle les parois des douilles (12, 14) sont munies de fenêtres radiales (16).

13. Cage spinale selon l'une des revendications 1 à 12, dans laquelle les douilles externes et internes (14, 12) sont de section circulaire.

14. Cage spinale selon l'une des revendications 1 à 13, dans laquelle les boulons filetés (42, 44) comportent une tête et une tige, la tige comportant une partie filetée (48) et une partie cylindrique (46) disposée entre ladite partie filetée et la tête, et étant d'un diamètre correspondant globalement au diamètre des trous radiaux (18, 20) de la douille externe (12).

15. Combinaison d'un outil et de la cage spinale selon l'une des revendications 8 à 14, dans laquelle l'outil a un manche oblong (56) présentant une poignée (54) à l'une de ses extrémités et une extension filetée (60) à l'autre extrémité.

16. Combinaison selon la revendication 15, dans laquelle une douille (62) est montée en rotation sur ladite extension filetée (68) afin de coopérer avec une épaule (68) dudit manche oblong (56) à une d'extrémité éloigné de celui-ci de ladite extension filetée (60), et comprend une cavité concave (66) d'une forme conforme avec respectivement la forme arrondie de ladite douille interne ou externe (14, 12).

17. Combinaison selon la revendication 16, dans laquelle ladite extension filetée (60) comprend une partie lisse (58) et ladite douille (62) a une partie filetée interne (64) espacée d'une extrémité libre de celle-ci, ladite partie filetée interne (64) étant hors de l'engagement avec les filets de ladite extension filetée (60) lorsque ladite douille (62) engage ladite épaule (68).
